# EUROPÄISCHE PATENTSCHRIFT

(11) **EP 0 289 977 B1**
(45) Veröffentlichungstag und Bekanntmachung des Hinweises auf die Patenterteilung: **15.09.1993**
(21) Anmeldenummer: 88107021.3
(22) Anmeldetag: 02.05.1988
(51) Int. Cl.: A61K 31/55, A61K 9/10, A61K 47/00

(54) **Orale Darreichungsformen mit verzögerter Abgabe**
Sustained-release oral administration forms
Formes d'administration orale à libération retardée

(30) Priorität: 04.05.1987 CH 1682/87
(43) Veröffentlichungstag der Anmeldung: 09.11.1988
(73) Patentinhaber: CIBA-GEIGY AG, 4002 Basel (CH)
(72) Erfinder: Khanna, Satish Chandra, Dr., CH-4103 Bottmingen (CH)
(74) Vertreter: Zumstein, Fritz, Dr.

(56) Entgegenhaltungen:
- DE-A- 2 950 476
- GB-A- 2 193 632
- US-A- 2 861 920
- CHEMICAL ABSTRACTS, Band 74, Nr. 16, 19. April 1971, Seite 222, Zusammenfassung Nr. 79592d, Columbus, Ohio, US; & JP-A-70 37 557 (SANKYO CO., LTD) 28-11-1970
- CHEMICAL ABSTRACTS, Band 101, Nr. 22, 26. November 1984, Seite 365, Zusammenfassung Nr. 198028k, Columbus, Ohio, US; E. LAINE et al.: "Formation of dihydrate from carbamazepine anhydrate in aqueous conditions"
- CHEMICAL ABSTRACTS, Band 95, Nr. 14, 5. Oktober 1981, Seite 364, Zusammenfassung Nr. 121077a, Columbus, Ohio, US; M. DAM et al.: "Carbamazep e: a clinical biopharmaceutical study"
- PHARMAZIE, Band 41, Nr. 1, Januar 1986, Seiten 61-62; H. KALA et al.: "Zum kristallografischen Verhalten von Carbamazepin-Zubereitungen unter Pressdruck"

## Beschreibung

Gegenstand der vorliegenden Erfindung ist eine stabile wässrige Suspension in Form eines Sirups mit verzögerter Abgabeeigenschaft von Carbamazepin-Dihydratkristallen, Verfahren zur Herstellung dieser oralen Darreichungsform und ihre Verwendung als Analgetikum und/oder Antikonvulsivum.

Carbamazepin, 5H-Dibenz[b,f]azepin-5-carboxamid (Tegretol®, Tegretal®: Ciba-Geigy) ist als Antikonvulsivum und als Analgetikum indiziert. Handelsübliche Darreichungsformen sind Tabletten mit 200 mg Wirkstoff und Sirupe mit 2%igem Wirkstoffgehalt. Generell haben flüssige orale Darreichungsformen wie Sirupe gegenüber Tabletten gewisse Vorteile, z.B. leichtere Einnehmbarkeit (besserer Geschmack) oder variable Dosierbarkeit. Daher sind Sirupe vor allem für Kinder geeignet.

Bei Beginn der Therapie mit Carbamazepin kann es zu gelegentlichen Nebenerscheinungen wie Appetitlosigkeit, Mundtrockenheit, Brechreiz, Diarrhoe, Obstipation, Kopfschmerzen, Schwindel, Somnolenz, Ataxie, Akkomodationsstörungen, Diplopie etc. kommen, siehe Schweizer Arzneimittelkompendium 1987, S. 1581 ff., Band 1, Documed Basel.

Beim handelsüblichen Sirup können diese ungünstigen Nebenerscheinungen der hohen Wirkstoffkonzentration im Plasma nach erfolgter Einnahme zugeschrieben werden. So werden maximale Wirkstoffkonzentrationen im Plasma bereits nach 1/2 bis 3 Stunden erreicht, siehe Schweizer Arzneimittelkompendium, loc. cit. Die schnelle Resorption des Wirkstoffs ist durch die Feinheit der im Sirup vorhandenen Carbamazepinkristalle bedingt:
Es ist bekannt, dass bei schwerlöslichen Wirkstoffen Lösungsgeschwindigkeit und als Folge davon Resorption und Uebertritt ins Blut erhöhte Werte annehmen können, wenn in der betreffenden Darreichungsform Wirkstoffe mit besonders kleiner Partikelgrösse vorliegen, siehe hierzu R. Voigt, Lehrbuch der Pharmazeutischen Technologie, Verlag Chemie, im folgenden "Voigt", Seite 636 unten (28.5.2.1): "Der Partikelgrösse kommt hiermit eine zentrale Bedeutung zu, da mit ihrer Verringerung und damit erfolgender Vergrösserung der spezifischen Oberfläche eine Erhöhung der Lösungsgeschwindigkeit und in Abhängigkeit davon der Resorption erreichbar ist."

Der handelsübliche Sirup enthält Carbamazepin-Hydratkristalle in Form von Nadeln mit einer Partikelgrösse kleiner als 10 µm. Die Anwesenheit von Carbamazepin-Hydratkristallen mit geringer Partikelgrösse erklärt sich durch das Herstellungsverfahren des Sirups:
Es ist bekannt, siehe J. Pharm. Soc. Jpn. No. 2, 184-190, 1984, dass wasserfreies Carbamazepin (amorph oder kristallin) in Kontakt mit Wasser ein Dihydrat bildet. Dieses Dihydrat liegt kristallin in Form von Nadeln vor, welche bis zu einer Teilchengrösse in Längsrichtung von ca. 100 bis 500 µm anwachsen können. Generell lassen sich nadelförmige Kristalle und insbesondere nadelförmige Kristalle dieser Grösse wegen ihrer Flocculationseigenschaften und/oder Sedimentationsneigung schlecht suspendieren, so dass man in einem separaten Verfahrensschritt ausgehend von wasserfreiem Carbamazepin eine wässrige Suspension aus Dihydratkristallen herstellt. Die nadelförmigen Kristalle müssen anschliessend nass, z.B. in einer Kolloidmühle, gemahlen werden. Beim Mahlvorgang lässt es sich nicht vermeiden, dass wechselnde Anteile des Mahlguts je nach Dauer und Intensität der Mahlung stets aus Bruchstücken mit einer Partikelgrösse kleiner als 10 µm bestehen.

Zur Zerkleinerung der grossen nadelförmigen Dihydratkristalle hat sich bisher nur das Nassmahlverfahren geeignet, da für trockene Mahlverfahren das Mahlgut eigens getrocknet werden muss. Die Trocknung stösst im Falle der Dihydratkristalle von Carbamazepin auf Schwierigkeiten, da sich dabei wieder wasserfreies Carbamazepin bildet. Bis jetzt stehen also nur aus nass gemahlenen, nadelförmigen Dihydratkristallen hergestellte, flüssige orale Darreichungsformen von Carbamazepin zur Verfügung.

Der vorliegenden Erfindung liegt die Aufgabe zugrunde, eine verbesserte orale Darreichungsform für Carbamazepin in Form einer stabilen wässrigen Suspension mit verzögerter Abgabeeigenschaft herzustellen.

Diese Aufgabenstellung wird durch die vorliegende Erfindung gelöst, welche einen Sirup enthaltend Carbamazepin-Dihydratkristalle in Würfelform oder würfelähnlicher Form und einer für die verzögerte Abgabe geeigneten Mindestpartikelgrösse von 10 µm bis 200 µm betrifft.

Die weiter vorn und im folgenden verwendeten allgemeinen Definitionen und Begriffe haben im Rahmen der Beschreibung der vorliegenden Erfindung vorzugsweise die folgenden Bedeutungen:
In einer stabilen wässrigen Suspension sind die Carbamazepin-Dihydratkristalle mehrere Tage bis Wochen dispergiert, ohne dass die vorzeitige Bildung von Sedimenten von Wirkstoffkristallen zu beobachten ist.

Die für die verzögerte Abgabe von Carbamazepin geeignete Partikelgrösse der Dihydratkristalle liegt in einem Bereich grösser als 10 µm bis 200 µm. Bevorzugt ist der Bereich grösser als 20 µm und kleiner als 100 µm. Es liegen mindestens 90 % der Dihydratkristalle in dem Bereich von 10 µm bis 200 µm und mindestens 80 % der Dihydratkristalle in dem Bereich von 20 µm bis 100 µm vor.

Die Partikelgrösse gibt einen ungefähren Wert an, der durch Messung der Durchmesser von Carbamazepin-Dihydratkristallen bestimmbar ist.

Zur Bestimmung dieses Werts eignen sich die bekannten Verfahren der Teilchengrössenanalytik, z.B. mikroskopische Methoden mit dem Lichtmikroskop, Teilchengrössenmessung mit Licht, z.B. Streulichtverfahren oder turbidimetrische Verfahren, Sedimentationsverfahren, z.B. Pipettieranalyse mit der Andreasenpipette, Sedimentationswaage, Photosedimentometer oder Sedimentation im Fliehkraftfeld, Impulsverfahren, z.B. mit Coulter Counter, oder Sichtung durch Schwer-oder Fliehkraft. Diese Methoden sind u.a. in Voigt auf den Seiten 64-79 beschrieben.

Vorzugsweise wird die Partikelgrösse aus einer Probe von Dihydratkristallen bestimmt, indem man zunächst eine repräsentative Auswahl, z.B. 90 % der Kristalle einer Probe, trifft, wobei man besonders grosse und kleine Kristalle eliminiert und von dieser durch Eliminierung der Extremwerte bereinigten Probe nach einem der genannten Verfahren einen mittleren Teilchendurchmesser mit Standardabweichung bestimmt. Bei den angegebenen Partikelgrössen beträgt die positive und negative Standardabweichung ca. 1-10 % vom gemittelten Wert.

Der erfindungsgemässe Sirup zeichnet sich durch besonders gute Verträglichkeit und hohe Lagerstabilität aus. So wird über längere Zeiträume von Wochen und Monaten keine Sedimentation von Wirkstoffkristallen beobachtet. Insbesondere ist überraschend, dass keine Bildung und Agglomeration von nadelförmigen Dihydratkristallen zu beobachten ist, was die Fliesseigenschaften und damit die Dosierung nachteilig verändern könnte. Durch die Verzögerung der Wirkstoffabgabe aus Kristallen von mehr als 10 µm, insbesondere mehr als 20 µm, Partikelgrösse, wird eine besonders gleichförmige Resorption des Wirkstoffs und Wirksamkeit auf therapeutischem Niveau unter Vermeidung der erhöhten Initialdosis erreicht.

Die erfindungsgemässe stabile wässrige Suspension wird hergestellt, indem man eine wässrige Dispersion mit einem geeigneten Schutzkolloid, welches das Wachstum von Carbamazepin-Hydratformen in Wasser in geeigneter Form und Grösse beeinflusst, herstellt und zu dieser Dispersion wasserfreies Carbamazepin hinzusetzt und die erhältliche Suspension zu einem Sirup weiterverarbeitet.

Dieses Verfahren hat wesentliche Vorteile, dass sich beispielsweise Sirupe herstellen lassen, worin ein besonders hoher Anteil der Wirkstoffmenge, z.B. mehr als 90 % Carbamazepindihydrat, in Form von Würfeln oder in würfelähnlicher Form im günstigen Partikelgrössenbereich von mehr als ca. 10 µm bis 200 µm vorliegt und sich insbesondere das technisch aufwendige und wegen der Bildung von Partikeln kleiner als 10 µm ungünstig erwiesene Nassmahlverfahren vermeiden lässt.

Der Begriff "wässrige Dispersion" umfasst wässrige Phasen, worin das Schutzkolloid in Abhängigkeit von seiner Löslichkeit in Wasser in suspendierter, kolloid-disperser oder gelöster Form vorliegt.

Das wasserfreie Carbamazepin, welches zur Herstellung der wässrigen Suspension unter Bildung der Hydratformen verwendet wird, besteht vorzugsweise aus amorphen oder kristallinen Formen beliebiger Partikelgrösse.

Schutzkolloide, welche das Wachstum von Carbamazepin-Hydratformen, insbesondere Dihydratkristallen in Form von Würfeln oder würfelähnlicher Form beeinflussen, verhindern die Bildung von Hydratkristallen, insbesondere Dihydratkristallen, mit einer Partikelgrösse von mehr als 200 µm. Vor allem fördern überraschenderweise solche Schutskolloide die Bildung von Würfelformen oder würfelähnlichen Formen und inhibieren die Bildung von nachteiligen Kristallformen, die zur Herstellung von Suspensionen ungeeignet sind, z.B. grösseren Dihydratkristallen in Nadelform. Wie weiter vorn erwähnt, ist die Bildung von grösseren nadelförmigen Dihydratkristallen aus wasserfreien Modifikationen oder amorphen Teilchen für die Herstellung von Suspensionen nachteilig, da die nadelförmigen Kristalle zu Agglomeraten und Sedimentation neigen und daher nass gemahlen werden müssen. Ausserdem haben Suspensionen mit nadelförmigen Dihydratkristallen schlechte Fliesseigenschaften.

Solche Schutzkolloide sind insbesondere wasserlösliche Polymere aus aliphatischen oder zyklischen Vinylamiden, z.B. Poly-N-vinylmethylacetamid, -ethylacetamid, -methylpropionamid, -ethylpropionamid, -methylisobutyramid, -2-pyrrolidon, -2-piperidon, -epsilon-caprolactam, -5-methyl-2-pyrrolidon oder -3-methyl-2-pyrrolidon, insbesondere Poly-N-vinylpyrrolidon mit einer mittleren Molmasse von ca. 10 000-360 000, oder Copolymere aus den genannten wasserlöslichen Polymeren aus aliphatischen oder zyklischen Vinylamiden, z.B. Poly-N-vinylpyrrolidon, mit wasserlöslichem Polyvinylacetat oder Polyvinylakohol mit unterschiedlichem Acetatgehalt, z.B. Polyvinylacetat mit einer Molmasse von ca. 5 000 bis 400 000 oder Polyvinylalkohl mit einem Hydrolysegrad von ca. 85-98 % und einem Polymerisationsgrad von ca. 500-2 500.

Als Schutzkolloide sind insbesondere Zusätze wie Poly-N-vinylpyrrolidon mit einer mittleren Molmasse von ca. 2500 bis 360 000, z.B. Kollidon® (BASF) Plasdone®, Peristone® (General Aniline) oder Luviskol® (BASF) mit folgenden Eigenschaften geeignet:
Löslich in Wasser, Ethanol, Methanol, Isopropanol, Glycerin, Propylenglycol, Methylenchlorid, unlöslich in Ether, Kohlenwasserstoffen, stark hygroskopisch (Wasseraufnahme von ca. 33 % ab ca. 70 % rel. Luftfeuchtigkeit), siehe Pharmazeutische Technologie, Sucker H. et al., Thieme Verlag Stuttgart 1978, Seite 339.

Ebenfalls bevorzugt ist das Vinylpyrrolidon-Vinylacetat-Copolymerisat mit einem Monomerenverhältnis Vinylpyrrolidon zu Vinylacetat von ca. 60:40 (Gew.-%) mit folgenden Eigenschaften:
Reinheit: 95 % (Rest Wasser), unlöslich in Ether, aliphatischen Kohlenwasserstoffen, sehr gut löslich in Wasser, Ethyl- und Isopropylalkohol, Methylenchlorid, Glycerin und 1,2-Propylenglycol, pH-Wert einer 10%igen wässrigen Lösung: 3-5, Viskosität (10%ige wässrige Lösung): 5 mPas, siehe H.P. Fiedler, Lexikon der Hilfsstoffe, Editio Cantor 1982.

Vinylpyrrolidon-Vinylacetat-Copolymerisate sind bekannt und/oder auf an sich bekannte Weise in beliebigem Mischungsverhältnis der Monomeren herstellbar. Das bevorzugte 60:40-Copolymerisat ist z.B. kommerziell unter der Bezeichnung Kollidon® VA 64 (BASF) erhältlich.

Die erfindungsgemässen Sirupe werden vorzugsweise hergestellt, indem man eine bestimmte Menge des Schutzkolloids in einem vorgegebenen Volumen Wasser dispergiert, so dass man zunächst eine ca. 0,1-20%ige, vorzugsweise eine ca. 1,0-5,0%ige, wässrige Dispersion erhält, die Dispersion gegebenenfalls sterilfiltriert und die gewünschte Menge an Carbamazepin (wasserfrei) hinzusetzt. Die Suspension kann anschliessend unter milden Bedingungen, vorzugsweise bei 5° bis ca. 80°, gerührt und zu Sirupen weiterverarbeitet werden.

In der wässrigen Suspension bilden sich würfelförmige oder würfelähnliche Dihydratkristalle mit einer durchschnittlichen Partikelgrösse grösser als ca. 10 µm und kleiner als ca. 200 µm. Dabei kann nach diesem Verfahren ein Anteil von mehr als 90 % an Kristallen mit dieser vorteilhaften Partikelgrösse gebildet werden.

Bei der Weiterverarbeitung der Suspension zu Sirupen, bedient man sich der üblichen galenischen Verfahren wie sie z.B. Hagers Handbuch der Pharmazeutischen Praxis, Springer Verlag 1971, Band VII, Teil A, Seiten 640-644, oder in Remington's Pharmaceutical Sciences, Mack 1985, Seiten 1500-1503, beschrieben sind.

Dabei können der wässrigen Suspension enthaltend die Carbamazepindihydratkristalle und Schutzkolloide für orale Formulierungen geeignete weitere Hilfsstoffe wie viskositätserhöhende Stoffe, Netzmittel, Konservierungsmittel, Antioxydantien, Farbstoffe, Geschmackkorrigenzien (Aromen), Zucker und/oder Süssstoffe (Sirupgrundlage) zugesetzt werden. Es können aber auch die Carbamazepindihydratkristalle durch Filtration vom Schutzkolloid getrennt werden. Die Carbamazepindihydratkristalle mit dem vorteilhaften Partikelgrössenbereich von ca. 10 µm bis 200 µm werden anschliessend in Wasser resuspendiert. Dieser Suspension kann man die Hilfsstoffe anschliessend zusetzen. Man kann aber auch die filtrierten Dihydratkristalle in der Sirupgrundlage suspendieren.

Viskositätserhöhende Stoffe sind z.B. anorganische Stabilisatoren von Suspensionen, z.B. kolloidale Silicate mit hohem Aluminium- und Magnesiumanteil wie Bentonite, Veegum oder Gelwhite, kolloidale Kieselsäure, z.B. Aerosil® (Degussa), Cabosil® (Cabot), organische Stabilisatoren, z.B. Quellmittel wie Alginate, z.B. Natriumalginat, Kaliumalginat oder Propylenglycolalginat, Gummi arabicum, Tragant, Karaya-Gummi, Sterculia-Gummi, Carageenan, Guar-Gummi oder Agar, synthetische oder halbsynthetische Quellmittel, z.B. 1,2-Epoxidpolymere, insbesondere Ethylenoxidhomopolymere mit einem Polymerisationsgrad von ca. 2 000-100 000, welche z.B. unter dem Handelsnamen Polyox® (Union Carbide) bekannt sind, quellfähige Celluloseether, z.B. Methyl- oder Ethylcellulose, Hydroxypropylcellulose, Hydroxypropylmethylcellulose, Methyl-oder Ethylhydroxyethylcellulose, Hydroxyethylcellulose oder Carboxymethylcellulose, mikrokristalline Cellulose oder zusätzliche Mengen an Polyvinylpyrrolidon.

Geeinete Netzmittel sind z.B. Sulfosuccinate wie Dihexyl-, Dioctyl-oder Diamylsulfosuccinat, Sulfonate oder Sulfate, z.B. Na-Alkylnaphthalinsulfonat, Fettalkoholsulfonate oder Fettalkoholpolyglycolethersulfate, Fettsäurepolyglycolester, z.B. Polyethylenglycolstearate, Polyglycolester von C₈-C₁₈-Fettsäuren, Fettalkoholpolyglycolether, z.B. Lauryl-, Cetyl-, Stearyl- oder Oleylalkoholpolyglycolether oder Cetylstearylalkoholpolyglycolether, Polyfettsäureester-polyglycolether, z.B. Polyethylenglycolsorbitanmonolaurat, -monopalmitat, -monostearat oder -monooleat, Glycerinfettsäureesterpolyglycolether oder Pentaerythritfettsäurepolyglycolether, Saccharoseester, z.B. Saccharosedistearat, Saccharosemono- und -distearat oder Saccharosemonopalmitat, Ethoxylierte pflanzliche Oele, z.B. ethoxyliertes Rizinusöl oder hydriertes und ethoxyliertes Rizinusöl, oder Blockpolymerisate wie Polyoxyethylen-Polyoxypropylen-Polymere.

Geeignete Konservierungsmittel sind z.B. Benzoesäure, Na-Benzoat, Sorbinsäure, z.B. Na- oder K-Sorbat, p-Hydroxybenzoesäuremethylester oder p-Hydroxybenzoesäurepropylester (Parabene) oder Chlorhexidindiacetat oder -digluconat.

Geeignete Antioxydantien sind z.B. Ascorbinsäure, Ascorbylpalmitat, Propylgallat, Nordihydroguajaretsäure (NDGA), Butylhydroxytoluol (BHT) oder Tocopherole, gegebenenfalls in Beimischung mit Synergisten wie Ascorbinsäure, Citronensäure, Citraconsäure, Phosphorsäure oder Weinsäure.

Die Verwendung von Farbstoffen kann der Hebung des Ausehens als auch der Kennzeichnung des Präparates dienen. Geeignete für die Verwendung in der Pharmazie zugelassene Farbstoffe sind z.B. Indigotin I (blau), Amaranth (rot), Gelborange S (orange) oder Tartrazin XX (gelb).

Zucker und Süssstoffe, welche als Sirupgrundlage dienen können, sind z.B. Saccharose, Xylitol, D-Xylose, Maltose, D-Glucose, Sorbitol, Glycerin, Mannitol, Dulcitol oder Lactose sowie Saccharin-Na, Dulcin, Ammoniumglycyrrhizinat oder Natriumcyclamat.

Die erfindungsgemässen oralen Darreichungsformen besitzen wertvolle pharmakologische Eigenschaften und können daher zur Behandlung von starken Schmerzzuständen und Konvulsionen verschiedenartiger Genese, z.B. Behandlung der Epilepsie, verwendet werden. Die Verwendung dieser oralen Darreichungsformen zur Behandlung solcher Krankheiten, insbesondere der Epilepsie, ist ebenfalls Gegenstand der vorliegenden Erfindung.

### Beispiel 1:

### a) Herstellung einer Wirkstoffzubereitung:

In 100 ml entionisiertem Wasser werden 1,0 g Vinylpyrrolidon-Vinylacetat-Copolymerisat (Kollidon® VA 64-BASF) gelöst und dazu 10,0 g wasserfreies Carbamazepin gegeben. Man dispergiert den Wirkstoff durch Rühren der Suspension. Es bilden sich würfelförmige und würfelähnliche Dihydratkristalle mit einer Partikelgrösse von ca. 10-200 µm.

### b) Herstellung eines Sirups:

Zu der gemäss a) erhältlichen Suspension werden folgende Hilfsstoffe gegeben:

| | |
|---|---|
| Mikrokristalline Cellulose (Avicel® RC 59) | 5,00 g |
| Carboxymethylcellulose-Natrium | 1,00 g |
| Na-Sacharin | 1,00 g |
| Methylparaben | 0,60 g |
| Propylparaben | 0,20 g |
| Entionisiertes Wasser ad | 500,00 ml |

Der Sirup kann anschliessend in 50 ml oder 100 ml Flaschen abgefüllt werden.

### Beispiel 2:

Analog Beispiel 1a) werden wässrige Suspensionen enthaltend 10 g Carbamazepin-Dihydratkristalle durch Zusatz von 2,0 g Poly-N-vinylpyrrolidon (Kollidon® K-30 oder Kollidon® K-90), oder 1,0 g Vinylpyrrolidon-Vinylacetat-Copolymerisat (Kollidon® VA 64) und 0,5 g Polyethylenglycol mit einem Polymerisationsgrad von ca. 2000-100'000 oder 2,0 g Poly-N-vinylpyrrolidon (Kollidon® K-30 oder Kollidon® K-90) und 0,5 g Polyethylenglycol mit einem Polymerisationsgrad von ca. 2000-100'000 hergestellt, welche sich zur Herstellung von Sirupen verwenden lassen.

## Patentansprüche (Patentansprüche für folgende(n) Vertragsstaat(en): AT, BE, CH, DE, FR, GB, IT, LI, LU, NL, SE)

1. Stabile wässrige Suspensionen in Form von Sirupen mit verzögerter Abgabeeigenschaft von Carbamazepin-Dihydratkristallen in Form von Würfeln oder würfelähnlicher Form mit einer Partikelgrösse von 10 µm bis 200 µm.

2. Verfahren zur Herstellung einer stabilen wässrigen Suspension gemäss Anspruch 1, dadurch gekennzeichnet, dass man eine wässrige Dispersion mit einem geeigneten Schutzkolloid, welches das Wachstum von Carbamazepin-Hydratformen in Wasser in geeigneter Form und Grösse beeinflusst, herstellt und zu dieser Dispersion wasserfreies Carbamazepin hinzusetzt und die erhältliche Suspenion zu einem Sirup weiterverarbeitet.

3. Verfahren gemäss Anspruch 2, dadurch gekennzeichnet, dass man eine Dispersion mit einem geeigneten Schutzkolloid, welches das Wachstum von Carbamazepin-Hydratformen in Wasser in Form von Würfeln oder in würfelähnlicher Form beeinflusst, herstellt.

4. Verfahren nach Anspruch 2 oder 3, dadurch gekennzeichnet, dass man als Schutzkolloid Poly-N-vinylpyrrolidon und gegebenenfalls Polyethylenglycol hinzusetzt.

5. Verfahren nach Anspruch 2 oder 3, dadurch gekennzeichnet, dass man als Schutzkolloid Vinylpyrrolidon-Vinylacetat-Copolymerisat mit einem Monomerenverhältnis Vinylpyrrolidon zu Vinylacetat von ca. 60:40 (Gew.-%) hinzusetzt.

6. Verwendung eines Schutzkolloids, welches das Kristallwachstum von Carbamazepin-Hydratformen in Wasser in Form von Würfeln oder in würfelähnlicher Form beeinflusst, zur Herstellung von stabilen wässrigen Suspensionen mit verzögerter Abgabeeigenschaften von Carbamazepin.

## Patentansprüche (Patentansprüche für folgende(n) Vertragsstaat(en): ES, GR)

1. Verfahren zur Herstellung einer stabilen wässrigen Suspension mit verzögerter Abgabeeigenschaft von Carbamazepin, dadurch gekennzeichnet, dass man eine wässrige Dispersion mit einem geeigneten Schutzkolloid, welches das Wachstum von Carbamazepin-Hydratformen in Wasser in Form von Würfeln oder in würfelähnlicher Form mit einer Partikelgrösse von ca. 10 µm bis ca. 200 µm beeinflusst, herstellt und zu dieser Dispersion wasserfreies Carbamazepin hinzusetzt und die erhältliche Suspenion zu einem Sirup weiterverarbeitet.

2. Verfahren gemäss Anspruch 1, dadurch gekennzeichnet, dass man eine bestimmte Menge des Schutzkolloids in einem vorgegebenen Volumen Wasser dispergiert, die Dispersion gegebenenfalls sterilfiltriert und die gewünschte Menge an Carbamazepin (wasserfrei) hinzusetzt.

3. Verfahren nach Anspruch 1 oder 2, dadurch gekennzeichnet, dass man als Schutzkolloid Poly-N-vinylpyrrolidon und gegebenenfalls Polyethylenglycol hinzusetzt.

4. Verfahren nach Anspruch 1 oder 2, dadurch gekennzeichnet, dass man als Schutzkolloid Vinylpyrrolidon-Vinylacetat-Copolymerisat und gegebenenfalls Polyethylenglycol hinzusetzt.

5. Verfahren nach Anspruch 4, dadurch gekennzeichnet, dass man als Schutzkolloid Vinylpyrrolidon-Vinylacetat-Copolymerisat mit einem Monomerenverhältnis Vinylpyrrolidon zu Vinylacetat von ca. 60:40 (Gew.-%) hinzusetzt.

## Claims (Claims for the following Contracting State(s): AT, BE, CH, DE FR, GB, IT, LI, LU, NL, SE)

1. A stable aqueous suspension in the form of a syrup, having a delayed release characteristic, of cubic or cuboid carbamazepine dihydrate crystals having a particle size of from 10 µm to 200 µm.

2. A process for the manufacture of a stable aqueous suspension according to claim 1, which comprises preparing an aqueous dispersion with a suitable protective colloid which influences the growth in water of carbamazepine hydrate forms of suitable shape and size and adding anhydrous carbamazepine to this dispersion and processing the obtainable suspension further to a syrup.

3. A process according to claim 2, which comprises preparing a dispersion with a suitable protective colloid which influences the growth in water of cubic or cuboid carbamazepine hydrate forms.

4. A process according to either claim 2 or claim 3, which comprises adding poly-N-vinylpyrrolidone and optionally polyethylene glycol as the protective colloid.

5. A process according to either claim 2 or claim 3, which comprises adding vinylpyrrolidone/vinyl acetate copolymer having a monomer ratio of vinylpyrrolidone to vinyl acetate of approximately 60:40 (% by weight) as the protective colloid.

6. The use of a protective colloid which influences the crystal growth in water of cubic or cuboid carbamazepine hydrate forms for the manufacture of stable aqueous suspensions, having delayed release characteristics, of carbamazepine.

## Claims (Claims for the following Contracting State(s): ES, GR)

1. A process for the manufacture of a stable aqueous suspension, having a delayed release characteristic, of carbamazepine, which comprises preparing an aqueous dispersion with a suitable protective colloid which influences the growth in water of cubic or cuboid carbamazepine hydrate forms having a particle size of from approximately 10 µm to approximately 200 µm and adding anhydrous carbamazepine to this dispersion and processing the obtainable suspension further to a syrup.

2. A process according to claim 1, which comprises dispersing a specific amount of the protective colloid in a predetermined volume of water, optionally sterile-filtering the dispersion and adding the desired amount of carbamazepine (anhydrous).

3. A process according to either claim 1 or claim 2, which comprises adding poly-N-vinylpyrrolidone and optionally polyethylene glycol as the protective colloid.

4. A process according to either claim 1 or claim 2, which comprises adding vinylpyrrolidone/vinyl acetate copolymer and optionally polyethylene glycol as the protective colloid.

5. A process according to claim 4, which comprises adding vinylpyrrolidone/vinyl acetate copolymer having a monomer ratio of vinylpyrrolidone to vinyl acetate of approximately 60:40 (% by weight) as the protective colloid.

## Revendications (Revendications pour l'(les) Etat(s) contractant(s) suivant(s): AT, BE, CH, DE, FR, GB, IT, LI, LU, NL, SE)

1. Suspensions aqueuses stables sous forme de sirops ayant une propriété de libération retardée de cristaux de dihydrate de carbamazépine sous une forme cubique ou sous forme cuboïde ayant une taille de particules de 10 µm à 200 µm.

2. Procédé pour la préparation d'une suspension aqueuse stable selon la revendication 1, caractérisé en ce qu'on prépare une dispersion aqueuse avec un colloïde protecteur approprié, qui influence la croissance des formes hydratées de carbamazépine dans l'eau dans une forme et une taille appropriées, et on ajoute à cette dispersion de la carbamazépine anhydre et on transforme la suspension obtenue en un sirop.

3. Procédé selon la revendication 2, caractérisé en ce qu'on prépare une dispersion avec un colloïde protecteur approprié, qui influence la croissance des formes hydratées de carbamazépine dans l'eau dans une forme cubique ou en une forme cuboïde.

4. Procédé selon l'une des revendications 2 ou 3, caractérisé en ce qu'on ajoute comme colloïde protecteur de la poly-N-vinylpyrrolidone et éventuellement du polyéthylèneglycol.

5. Procédé selon l'une des revendications 2 ou 3, caractérisé en ce qu'on ajoute comme colloïde protecteur un copolymère de vinylpyrrolidone-acétate de vinyle ayant un rapport des monomères vinylpyrrolidone/acétate de vinyle d'environ 60:40 (% en poids).

6. Utilisation d'un colloïde protecteur, qui influence la croissance des formes hydratées de carbamazépine dans l'eau dans une forme cubique ou une forme cuboïde, pour la préparation de suspensions aqueuses stables ayant des propriétés de libération retardée de la carbamazépine.

## Revendications (Revendications pour l'(les) Etat(s) contractant(s) suivant(s): ES, GR)

1. Procédé pour la préparation d'une suspension aqueuse stable ayant une propriété de libération retardée de la carbamazépine, caractérisé en ce qu'on prépare une dispersion aqueuse avec un colloïde protecteur approprié, qui influence la croissance des formes hydratées de carbamazépine dans l'eau dans une forme cubique ou une forme cuboïde ayant une taille de particules de 10 µm à 200 µm, et on ajoute à cette dispersion de la carbamazépine anhydre et on transforme la suspension obtenue en un sirop.

2. Procédé selon la revendication 1, caractérisé en ce qu'on disperse une quantité déterminée du colloïde protecteur dans un volume d'eau prédéfini, on soumet éventuellement la dispersion à une filtration stérile et on ajoute la quantité souhaitée de carbamazépine (anhydre).

3. Procédé selon l'une des revendications 1 ou 2, caractérisé en ce qu'on ajoute comme colloïde protecteur de la poly-N-vinylpyrrolidone et éventuellement du polyéthylèneglycol.

4. Procédé selon l'une des revendications 1 ou 2, caractérisé en ce qu'on ajoute comme colloïde protecteur un copolymère de vinylpyrrolidone-acétate de vinyle et éventuellement du polyéthylèneglycol.

5. Procédé selon la revendication 4, caractérisé en ce qu'on ajoute comme colloïde protecteur un copolymère de vinylpyrrolidone-acétate de vinyle ayant un rapport des monomères vinylpyrrolidone/acétate de vinyle d'environ 60:40 (% en poids).
